# EUROPÄISCHE PATENTSCHRIFT

(11) **EP 4 061 440 B1**
(45) Veröffentlichungstag und Bekanntmachung des Hinweises auf die Patenterteilung: **09.07.2025**
(21) Anmeldenummer: 19805652.5
(22) Anmeldetag: 18.11.2019
(51) Int. Cl.: A61M 1/00, B01D 19/00

(54) **SAUGKOPF ZUM SCHONENDEN ABSAUGEN THIXOTROPER FLÜSSIGKEITEN**
SUCTION HEAD FOR THE GENTLE SUCTIONING OF THIXOTROPIC FLUIDS
TÊTE D'ASPIRATION POUR L'ASPIRATION DOUCE DE FLUIDES THIXOTROPES

(43) Veröffentlichungstag der Anmeldung: 28.09.2022
(73) Patentinhaber: Georg-August-Universität Göttingen Stiftung Öffentlichen Rechts, Universitätsmedizin, 37075 Göttingen (DE)
(72) Erfinder: FRIEDRICH, Martin, 37120 Bovenden (DE)
(74) Vertreter: REHBERG HÜPPE + PARTNER
(86) Internationale Anmeldenummer: PCT/EP2019/081620
(87) Internationale Veröffentlichungsnummer: WO 2021/098939

(56) Entgegenhaltungen:
- DE-A1- 19 650 407
- KR-B1- 101 843 145
- KR-B1- 101 981 324
- US-A- 5 824 212
- US-A1- 2017 224 887

## Beschreibung

### TECHNISCHES GEBIET DER ERFINDUNG

Die Erfindung betrifft einen Saugkopf zum Absaugen einer organische Bestandteile umfassenden Flüssigkeit. Insbesondere betrifft die Erfindung einen Saugkopf zum Absaugen einer organische Bestandteile umfassenden Flüssigkeit mit einer einen Hauptkanal des Saugkopfs definierenden Innenoberfläche, wobei sich der Hauptkanal längs einer Hauptachse des Saugkopfs zu einem Absauganschluss des Saugkopfs hin erstreckt, und mit in den Saugkopf eintretenden und durch die Innenoberfläche in den Hauptkanal einmündenden Sauglöchern.

Bei der abzusaugenden organische Bestandteile umfassenden Flüssigkeit kann es sich insbesondere um eine biologische Flüssigkeit, wie beispielsweise Blut, aber auch um jede andere Körperflüssigkeit oder eine andere Flüssigkeit mit biologischen oder anderen organischen Bestandteilen handeln. Dabei schließen biologische Bestandteile zum Beispiel lebende Zellen aber auch große organische Moleküle und Komplexe, wie Nukleinsäureketten und Eiweiße, ein. Die organischen Bestandteile können in der abzusaugenden Flüssigkeit gelöst oder suspendiert sein.

Beim Absaugen von Blut aus Operationsfeldern, um dieses an den Patienten zurückzugeben, an dem die jeweilige Operation durchgeführt wird, treten verschiedene Problematiken auf. Hierzu zählt das Vermischen des Bluts mit Umgebungsluft in Form von Luftblasen, das Schädigen von Bestandteilen des Bluts, inklusive und insbesondere der roten Blutkörperchen, weißen Blutkörperchen und Thrombozyten, durch Scherkräfte und das Festsaugen eines zum Absaugen verwendeten Saugkopfs an angrenzendem Gewebe des Patienten. Mit dem Blut vermischte Umgebungsluft muss wieder entfernt werden, bevor das Blut an den Patienten zurückgegeben werden darf, um Luftembolien zu verhindern. Wenn durch Scherkräfte geschädigtes Blut an einen Patienten zurückgegeben wird, kann dies zu nicht kalkulierbaren Schädigungen führen, u. a. Nierenversagen, Lungenschädigung, Thrombosen, Wundheilungsstörungen, systemische Inflammationsreaktionen. Das Festsaugen eines Saugkopfs an Gewebe ist mit der Gefahr der Schädigung dieses Gewebes verbunden. Zudem können bei einem sich intermittierend festsaugenden Saugkopf sehr große Scherkräfte auf das abgesaugte Blut auftreten, während ein dauerhaft festgesaugter Saugkopf funktionslos ist.

Entsprechende Problematiken treten beim Absaugen anderer komplexere organische Bestandteile umfassenden Flüssigkeiten auf, wie u. a. unerwünschte Aktivierungen, Fehlaktivierungen, Strukturänderungen an Molekülen wie Faltungen, Denaturierungen, Zerfall und ähnliches.

### STAND DER TECHNIK

Aus der WO 2012/092948 A1 ist eine Absaugvorrichtung zur Absaugung von Blut aus Operationsfeldern bekannt. Sie weist einen Saugkopf mit einer frontseitigen Saugöffnung an seinem distalen Ende sowie mehreren seitlich angeordneten Saugöffnungen und eine mit dem Saugkopf verbundene Pumpe auf. Die Pumpe erzeugt einen Saug-Unterdruck; und mit einer Steuereinrichtung wird eine an den Saugöffnungen wirksame Saugleistung eingestellt. Die Steuereinrichtung ist mit einem Schallwellensensor verbunden, der durch den Saugkopf bei dessen Betrieb erzeugte Schallwellen und andere Schwingungen aufnimmt. Wenn die Steuereinrichtung mittels des Schallwellensensors ein charakteristisches Schallwellenmuster detektiert, das für ein schlürfendes Sauggeräusch steht, reduziert sie die Saugleistung an den Saugöffnungen, weil das schlürfende Sauggeräusch auf ungünstige Absaugbedingungen hinweist, die mit der Gefahr einer Schädigung von Blutbestandteilen verbunden ist. Zusätzlich ist die Steuereinrichtung mit einem Festsaug-Sensor verbunden, und beim Erkennen eines Festsaugens des Saugkopfs mit dem Festsaug-Sensor reduziert sie ebenfalls die Saugleistung an den Saugöffnungen.

Aus der US 2014/0276486 A1 ist eine Absaugvorrichtung für Blut aus Operationsfeldern mit einem Saugkopf bekannt. Der Saugkopf weist einen hohlen Hauptkörper mit einer Mehrzahl von Öffnungen auf, die eine Fluidkommunikation zwischen der Umgebung und einem Innenraum des hohlen Hauptkörpers ermöglichen. Der Saugkopf weist weiterhin einen zylindrischen Fortsatz auf, der von dem hohlen Hauptkörper absteht und an den eine Absaugleitung angeschlossen ist. Der Hauptkörper ist kapselförmig mit abgerundeten Enden. Eine Öffnung des Hauptkörpers ist an seinem distalen Ende vorgesehen, die anderen Öffnungen an seinem Umfang.

Aus der US 7,955,318 B1 ist ein medizinisches Saugsystem mit Saugeinrichtungen zum Entfernen von Material aus Körperhöhlen während medizinischer Prozeduren bekannt. Das System ist an eine Vakuumquelle mit großem Anschlussquerschnitt, wie beispielsweise den Anschluss eines Vakuumsammelbehälters anschließbar. Das System umfasst eine Reihe von austauschbaren Saugköpfen. Einer dieser Saugköpfe weist eine atraumatische Form zum Reduzieren von Gewebetrauma während der medizinischen Prozedur auf. Die atraumatische Form umfasst eine im Wesentlichen abgerundete Oberfläche, und dieser Saugkopf wird auch als Saugkomponente mit stumpfer Spitze bezeichnet. Der Saugkopf weist eine Mehrzahl von distalen Spitzeneinlässen und eine Mehrzahl von Seiteneinlässen auf, die zusammen ein feines siebartiges Saugfeld ausbilden.

Die WO 88/00481 A1 beschreibt eine chirurgische Saugvorrichtung mit einem perforierten Saugkopf zum Entfernen von chirurgischen Trümmern mit reduzierter Verstopfung und minimalem Trauma von angrenzendem Gewebe. Saugöffnungen sind derart an dem Saugkopf angeordnet, dass die Saugöffnungen, die unblockiert bleiben, wenn chirurgische Trümmer in anderen Saugöffnungen hängenbleiben, als Vakuummodulatoren wirken, die das Entfernen der Blockade erleichtern. Darüber hinaus ist die Wahrscheinlichkeit des Blockierens aller Saugöffnungen und damit des Ansaugens und Beschädigens von Gewebe reduziert. Konkret sind sowohl an einer Vorderseite als auch einer Rückseite des knollenförmigen Saugkopfs Sauglöcher vorgesehen, die zu einem Zentrum des knollenförmigen Saugkopfs hin verlaufen.

Aus der US 5,827,218 ist ein Saugkopf für eine chirurgische Spülvorrichtung bekannt. Der Saugkopf umfasst ein äußeres Rohr mit einem distalen Endbereich zur Kommunikation mit einem Operationsort und ein Innenrohr, das sich in dem Außenrohr erstreckt und ein offenes distales Ende zur Kommunikation mit dem Operationsort aufweist. Der Saugkopf ist ausgebildet, um während des Absaugens Turbulenz nahe empfindlicher Organe zu minimieren. Ein Turbulenz minimierender distaler Endbereich des äußeren Rohrs weist ein konvex abgerundetes Ende mit in Umfangsrichtung und axial beabstandeten Saugströmungslöchern durch die Wand des äußeren Rohrs in sein hohles Inneres auf. Die Saugströmungslöcher sind in sich axial erstreckenden, in Umfangsrichtung beabstandeten Reihen angeordnet, wobei jede Reihe ein nach vorne zeigendes Loch am gerundeten Ende und sechs sich radial erstreckende Löcher umfasst.

Aus der US 5,163,926 A ist eine Saug- und Mischvorrichtung zum Absaugen von Körperflüssigkeiten, insbesondere Blut, und gleichzeitigem Vermischen mit einem Antikoagulationsmittel bekannt. Die Vorrichtung weist neben einem an einer Unterdruckquelle angeschlossenen Saugrohr ein Zuführrohr für das Antikoagulationsmittel auf. Über einem gemeinsamen distalen Ende der beiden Rohre ist ein Saugkopf angeordnet, in dessen Innenraum neben den beiden Rohren mehrere Sauglöcher für die Körperflüssigkeit einmünden.

Aus der US 2017 / 0 224 887 A1 ist ein System zum Auftrennen eines Materialstroms bekannt, bei dem der Materialstrom von einem chirurgischen Instrument tangential in einen zylindermantelförmigen Hohlraum eingeleitet wird. Bezüglich seiner Zylinderachse ist der Hohlraum vertikal ausgerichtet. Am oberen Ende des Ringraums wird abgesaugt, und an seinem unteren Ende wird aus dem Strom abgetrenntes Material abgeführt.

Aus der DE 196 50 407 A1 ist eine Vorrichtung zum Trennen von Gas aus Blut, insbesondere in einem Blutstrom, welcher von einer Wundstelle eines Patienten abgesaugt wird, bekannt. Eine nicht rotierende Zentrifugierkammer hat von einem Bluteinlass am oberen Kammerende zu einem Blutauslass am unteren Kammerende eine trichterartig enger werdende Form. An den Blutauslass ist eine Saugvorrichtung nageschlossen, die einen um eine vertikale Rotationsachse rotierenden Blutstrom durch die Zentrifugierkammer hervorruft. Eine Strömungsrichtung des Bluteinlasses ist im Wesentlichen tangential zur Rotationsachse und gleichzeitig in Blutströmungsrichtung schräg von oben nach unten in die Zentrifugierkammer gerichtet, mit einem nach oben öffnenden Winkel zur Rotationsachse von weniger als 90°.

Aus der KR 101 981 324 B1 ist ein Saugkopf zum Absaugen unter anderem von Flüssigkeiten wie Speichel, Blut oder Wasser bekannt, der die Merkmale des Oberbegriffs des unabhängigen Patentanspruchs 1 aufweist. Dabei sind die Sauglöcher spiralförmig zu dem Hauptkanal hin ausgebildet. Weiterhin sind in einer Außenoberfläche des Saugkopfs spiralförmige Nuten ausgebildet, die in die spiralförmigen Sauglöcher übergehen. Durch Spiralform der Sauglöcher ist deren Durchtrittsfläche vergrößert, so dass ihr Bereich, der nicht durch Weichgewebe blockiert werden kann, vergrößert ist. Außerdem wird ein Spiralwirbel induziert, wenn Flüssigkeit in den Hauptkanal eingesaugt wird, sodass die Flüssigkeit schnell abgesaugt wird, während sie in dem Spiralwirbel rotiert wird.

Es ist bekannt, dass Blut keine sogenannte Newton'sche Flüssigkeit, sondern eine thixotrope Flüssigkeit ist, deren Viskosität bei höheren Scherkräften und Fließgeschwindigkeiten abnimmt.

### AUFGABE DER ERFINDUNG

Der Erfindung liegt die Aufgabe zugrunde, einen Saugkopf aufzuzeigen, der die eingangs geschilderten Problematiken beim Absaugen von organische Bestandteile umfassenden Flüssigkeiten und insbesondere von Blut aus Operationsfeldern minimiert.

### LÖSUNG

Die Aufgabe der Erfindung wird durch einen Saugkopf mit den Merkmalen des unabhängigen Patentanspruchs 1 gelöst. Die abhängigen Patentansprüche 2 bis 15 sind auf bevorzugte Ausführungsformen des erfindungsgemäßen Saugkopfs gerichtet.

### BESCHREIBUNG DER ERFINDUNG

Bei einem erfindungsgemäßen Saugkopf zum Absaugen einer organische Bestandteile umfassenden Flüssigkeit mit einer einen Hauptkanal des Saugkopfs definierenden Innenoberfläche, wobei sich der Hauptkanal längs einer Hauptachse des Saugkopfs zu einem Absauganschluss des Saugkopfs hin erstreckt, und mit in den Saugkopf eintretenden und durch die Innenoberfläche in den Hauptkanal einmündenden Sauglöchern sind Strömungsleiteinrichtungen des Saugkopfs derart ausgebildet, dass die Strömungsleiteinrichtungen einer durch einen Unterdruck in dem Absauganschluss hervorgerufen Strömung der durch die Sauglöcher eintretenden Flüssigkeit durch den Hauptkanal eine Rotationskomponente um die Hauptachse aufprägen. Mit anderen Worten führen die Strömungsleiteinrichtungen dazu, dass die Strömung der Flüssigkeit nicht nur längs der Hauptachse des Saugkopfs durch den Hauptkanal verläuft, sondern auch um diese Hauptachse herum. Unterstellt man, dass die Geschwindigkeit der Flüssigkeit längs der Hauptachse des Saugkopfs gleich bleibt, bedeutet die zusätzliche Rotationskomponente der Strömung eine von ihrem Betrag her größere absolute Geschwindigkeit der Strömung. Bei thixotropen Eigenschaften der organische Bestandteile umfassenden Flüssigkeit hat dies in vorteilhafter Weise eine reduzierte Viskosität der Flüssigkeit zur Folge. Der mit der Viskosität abnehmende viskose Strömungswiderstand der Flüssigkeit bedeutet nicht nur einen geringeren Fließwiderstand, sondern auch eine schnellere Abscheidbarkeit von Luftblasen aus der Flüssigkeit. Die Rotationskomponente der Strömung der Flüssigkeit um die Hauptachse und die damit verbundenen Zentrifugalkräfte können so groß werden, dass sich mit der Flüssigkeit durch die Sauglöcher in den Hauptkanal eingesaugte Luft im Bereich der Hauptachse konzentriert und von dort abgeführt werden kann, während sich die Flüssigkeit an der den Hauptkanal begrenzenden Innenoberfläche ansammelt und von dort getrennt von der Luft abgesaugt werden kann. Zudem können für die auf Spiralbahnen durch den erfindungsgemäßen Saugkopf strömende Flüssigkeit besonders gut jegliche Unstetigkeiten vermieden werden, die mit der Gefahr verbunden sind, Drucksprünge und/oder große Scherkräfte auf die organischen Bestandteile der Flüssigkeit hervorzurufen, die eine Schädigung dieser organischen Bestandteile zur Folge haben können.

Soweit hier von einem Unterdruck die Rede ist, ist damit ein Druck unterhalb des Drucks in der Umgebung des Saugkopfs gemeint.

Konkret kann der von der Strömung der Flüssigkeit durch den Hauptkanal zurückgelegte Weg aufgrund der Rotationskomponente um die Hauptachse um mindestens 50 % oder 100 % länger sein als die Erstreckung des Hauptkanals längs der Hauptachse. Der Weg der Strömung durch den Hauptkanal kann aber auch auf deutlich mehr als das Doppelte des Mindestwegs längs der Hauptachse, und konkret beispielsweise auf mindestens das Dreifache, das Fünffache oder sogar das Zehnfache verlängert sein. Nach oben ist der Verlängerung des von der Strömung der Flüssigkeit durch den Hauptkanal zurückgelegte Wegs durch die weiterhin vorhandenen Strömungskomponente längs der Hauptachse des erfindungsgemäßen Saugkopfs eine natürliche Obergrenze gesetzt. So kann der von der Strömung der Flüssigkeit durch den Hauptkanal zurückgelegte Weg aufgrund der Rotationskomponente um die Hauptachse kaum mehr als 500-mal länger sein als die Erstreckung des Hauptkanals längs der Hauptachse. Meistens wird er nicht mehr als 100-mal länger sein.

Die Rotationskomponente der Strömung um die Hauptachse kann durch verschiedene Maßnahmen hervorgerufen werden. So können die Strömungsleiteinrichtungen eines oder mehrere der folgenden Merkmale umfassen.

Einmündungen der Sauglöcher in den Hauptkanal können eine bezogen auf einen Kreisbogen um die Hauptachse tangentiale Richtungskomponente aufweisen. Die Einmündungen können exakt tangential zu dem Kreisbogen um die Hauptachse verlaufen oder neben der tangentialen Richtungskomponente eine axiale Richtungskomponente längs der Hauptachse, insbesondere zu dem Absauganschluss hin, aufweisen.

Eine Abzweigung des Absauganschlusses von dem Hauptkanal kann eine bezogen auf einen Kreisbogen um die Hauptachse tangentiale Richtungskomponente aufweisen. Auch diese tangentiale Richtungskomponente kann die einzige Richtungskomponente der Abzweigung des Absauganschlusses sein oder mit einer axialen Richtungskomponente kombiniert sein.

Eine Einmündung einer Einspritzdüse für eine Hilfsflüssigkeit in den Hauptkanal kann eine in Bezug auf einen Kreisbogen um die Hauptachse tangentiale Richtungskomponente aufweisen. Im Falle von Blut als abgesaugter Flüssigkeit kann die Hilfsflüssigkeit zum Beispiel eine Heparin-Lösung oder eine andere Flüssigkeit sein, die eingesetzt wird, um ein Koagulieren des Bluts zu verhindern oder dessen Fließeigenschaften zu verbessern. Auch für die Einmündung der Einspritzdüse für die Hilfsflüssigkeit gilt, dass diese ausschließlich die tangentiale Richtungskomponente aufweisen kann oder auch eine zusätzliche axiale Richtungskomponente. Eine automatische Dosierung der Hilfsflüssigkeit kann durch den an der Einmündung der Einspritzdüse in den Hauptkanal herrschenden Unterdruck bewirkt werden.

An der den Hauptkanal begrenzenden Innenoberfläche können spiralförmige Strömungsleitelemente angeordnet sein. Diese Leitelemente können die Form von radial nach innen vorstehenden und spiralförmig um die Hauptachse umlaufenden Rippen haben. Die Innenoberfläche kann weiterhin mit einem Lotus-Effekt versehen sein, der zur Folge hat, dass der Strömungswiderstand für die Flüssigkeit über die Innenoberfläche längs einer Spiralbahn um die Hauptachse geringer ist als parallel zu der Hauptachse. Neben den bisher beschriebenen passiven Maßnahmen kann die Strömungsleiteinrichtung auch aktive Maßnahmen umfassen, wie beispielsweise einen einen Teil der Innenoberfläche ausbildenden und um die Hauptachse herum hin und her drehangetriebenen Beschleunigungskörper. Ein solcher Beschleunigungskörper ruft eine Rotationskomponente der Strömung um die Hauptachse herum auf, wenn er mit unterschiedlichen Drehgeschwindigkeiten in der einen und der anderen Drehrichtung um die Hauptachse herum drehangetrieben wird und/oder wenn er eine beispielsweise schuppenförmig strukturierte Oberfläche aufweist. Eine weitere aktive Maßnahme ist ein in dem Hauptkanal angeordneter, um die Hauptachse herum kontinuierlich drehangetriebener Beschleunigungskörper. Ein solcher Beschleunigungskörper kann auch eine glatte Oberfläche aufweisen.

Der von der Strömung der Flüssigkeit durch den Hauptkanal aufgrund der Rotationskomponente um die Hauptachse zusätzlich zurückgelegte mit der Erstreckung des Hauptkanals längs der Hauptachse verglichene Weg kann zu dem Absauganschluss hin zunehmen. Eine solche Zunahme kann durch eine geringer werdende Steigung und/oder einen zunehmenden Durchmesser der Spiralbahnen der Strömung um die Hauptachse erreicht werden. Bei anderen Ausführungsformen des erfindungsgemäßen Saugkopfs nimmt der von der Strömung der Flüssigkeit durch den Hauptkanal aufgrund der Rotationskomponente um die Hauptachse zusätzlich zurückgelegte Weg gegenüber der Erstreckung des Hauptkanals längs der Hauptachse zu dem Absauganschluss hin hingegen ab. Dies kann durch eine größer werdende Steigung und/oder einen kleiner werdenden Durchmesser der Spiralbahnen um die Hauptachse erreicht werden.

Ein freier Strömungsquerschnitt des Saugkopfs längs der Strömung der Flüssigkeit zu dem Absauganschluss hin nimmt typischerweise ab, was eine Zunahme einer Strömungsgeschwindigkeit der Strömung zur Folge hat. Dabei kann sich die Abnahme des freien Strömungsquerschnitts des Saugkopfs auf die Sauglöcher beschränken, die trompetenförmig ausgebildet sein können, um die Flüssigkeit in den Sauglöchern sukzessive zu dem Hauptkanal hin zu beschleunigen. Die Abnahme des freien Strömungsquerschnitts längs der Strömung kann sich in den Hauptkanal hinein fortsetzen. Hier kann der freie Strömungsquerschnitt aber auch gleich bleiben oder wieder zunehmen, um die Strömungsgeschwindigkeit der abgesaugten Flüssigkeit einzustellen. In jedem Fall ist es bevorzugt, wenn der freie Strömungsquerschnitt einen stetigen Verlauf, d. h. keine sprunghaften Änderungen, aufweist. Vorzugsweise weist auch die Änderung des freien Strömungsquerschnitts einen stetigen Verlauf auf, d. h. dass auch die Ableitung des freien Strömungsquerschnitts nach dem Weg der Strömung keine Sprünge aufweist.

Die Sauglöcher können konkret jeweils eine von einer Außenoberfläche zu der Innenoberfläche des Saugkopfs hin abnehmende freie Querschnittsfläche aufweisen. Diese freie Querschnittsfläche der Sauglöcher kann von der Außenoberfläche bis zu der Innenoberfläche jeweils um mindestens 50 %, d. h. auf die Hälfte, abnehmen. Die Abnahme kann auch um mindestens 67 %, d. h. auf etwa ein Drittel, oder um mindestens 75 %, d. h. auf ein Viertel, erfolgen. Nach oben ist der Abnahme der freien Querschnittsfläche der Sauglöcher von der Außenoberfläche bis zu der Innenoberfläche durch die notwendigerweise verbleibende freie Querschnittsfläche eine natürliche Obergrenze gesetzt. So kann die Abnahme der freien Querschnittsfläche der Sauglöcher von der Außenoberfläche bis zu der Innenoberfläche kaum mehr 95 % betragen. Meistens wird er nicht mehr als 90 % betragen.

Bei den Sauglöchern ist es bevorzugt, wenn eines der Sauglöcher, das näher zu dem Absauganschluss hin in den Saugkopf eintritt, einen größeren Strömungswiderstand für die Flüssigkeit bis in den Hauptkanal aufweist als eines der Sauglöcher, das weiter weg von dem Absauganschluss in den Saugkopf eintritt. Wenn ein erfindungsgemäße Saugkopf in eine Lache der abzusaugenden Flüssigkeit eingetaucht wird, ist es häufig so, dass nur die am distalen Ende des Saugkopfs und damit weiter weg von dem Absauganschluss in den Saugkopf eintretenden Sauglöcher ganz in die Flüssigkeit eintauchen, während näher zu dem Absauganschluss hin in den Saugkopf eintretende Sauglöcher frei liegen. Um einen zumindest kompletten Kurzschluss der Sauglöcher an dem distalen Ende des Saugkopfs durch in die frei liegenden Sauglöcher eingesaugte Luft zu verhindern, nimmt der Strömungswiderstand der Sauglöcher bezogen auf den Ort ihres Eintritts in den Saugkopf zu dem distalen Ende des Saugkopfs hin vorzugsweise ab bzw. zu dem Absauganschluss hin vorzugsweise zu. Konkret kann dasjenige der Sauglöcher, das zu dem Absauganschluss hin am nächsten in den Saugkopf eintritt, verglichen mit dem Saugloch, das von dem Absauganschluss am weitesten entfernt in den Saugkopf eintritt, einen um mindestens 50 % erhöhten Strömungswiderstand aufweisen. Vorzugsweise ist der Strömungswiderstand um mindestens 100 %, d. h. auf mindestens das Doppelte, oder sogar um mindestens 200 %, d. h. auf mindestens das Dreifache, erhöht. Nach oben ist der Erhöhung des Strömungswiderstands durch die notwendigerweise verbleibende Funktion der betrachteten Sauglöcher eine natürliche Obergrenze gesetzt. So ist eine Erhöhung des Strömungswiderstands auf mehr als das 10-fache kaum sinnvoll. Meistens wird sie auf nicht mehr als das 5-fache erfolgen.

Bei dem erfindungsgemäßen Saugkopf kann die Innenoberfläche zumindest teilweise und/oder können die Sauglöcher mit einer den Überströmungswiderstand für die Flüssigkeit herabsetzenden Slipbeschichtung versehen sein. Geeignete Materialien für die Slipbeschichtung sind dem Fachmann bekannt. Sie bilden eine Oberfläche aus, an der auch eine Grenzschicht der Flüssigkeit aufgrund hoher Grenzflächenspannungen nicht anhaftet, sondern abgleitet.

Hierdurch wird der Strömungswiderstand, den die Flüssigkeit beim Durchströmen der Sauglöcher bzw. des Hauptkanals zu überwinden hat, signifikant reduziert.

Der erfindungsgemäße Saugkopf kann einen 3D-gedruckten Formkörper umfassen, um den Sauglöchern und auch dem Hauptkanal mit der ihn begrenzenden Innenoberfläche eine Form geben zu können, die sich nicht ohne weiteres beispielsweise durch ein spanabnehmendes oder abformendes Herstellungsverfahren ausbilden lässt. Die beim 3D-Drucken erzielbare Oberflächenqualität ist für den erfindungsgemäßen Saugkopf vielfach nicht ausreichend. Dies kann aber dadurch ausgeglichen werden, dass die Oberflächen des 3D-gedruckten Formkörpers mit einer zusammenhängenden glatten Beschichtung versehen werden. Eine derartige Beschichtung kann beispielsweise unter Eintauchen des Formkörpers in ein entsprechendes Beschichtungsmaterial und/oder Einsaugen eines entsprechenden Beschichtungsmaterials in den Formkörper ausgebildet werden.

Bei dem erfindungsgemäßen Saugkopf weist der Absauganschluss einen auf der Hauptachse an den Hauptkanal anschließenden inneren Teilanschluss und einen mit Abstand zu der Hauptachse durch die Innenoberfläche in den Hauptkanal einmündenden äußeren Teilanschluss auf. Wenn der Hauptkanal nicht vollständig mit der abzusaugenden Flüssigkeit gefüllt ist, sammelt sich zusammen mit dieser in den Saugkopf eingesaugte Luft im Bereich der Hauptachse an, während die auf den Spiralbahnen geführte Flüssigkeit über die den Hauptkanal begrenzende Innenoberfläche fließt. So kann über den inneren Teilanschluss gezielt die Luft abgeführt werden und über den äußeren Teilanschluss gezielt die Flüssigkeit abgesaugt werden. Dabei kann eine Umschalteinrichtung, die zwischen den beiden Teilanschlüssen umschaltet, so ausgebildet sein, dass sie beim Ansaugen von Luft durch den Saugkopf den auf der Hauptachse an den Hauptkanal angeschlossenen inneren Teilanschluss öffnet und den mit Abstand zu der Hauptachse durch die Innenoberfläche in den Hauptkanal einmündenden äußeren Teilanschluss schließt.

An seinem dem Absauganschluss gegenüberliegenden Ende ist der Hauptkanal des erfindungsgemäßen Saugkopfs typischerweise mindestens zu einem Drittel oder zur Hälfte geschlossen, um die Ausbildung einer primär axialen Durchströmung des Hauptkanals zu verhindern. Der Hauptkanal kann zu diesem Zweck an seinem dem Absauganschluss gegenüberliegenden Ende auch zu mindestens zwei Dritteln oder zu drei Vierteln oder gar vollständig geschlossen sein. Solange der Hauptkanal an seinem dem Absauganschluss gegenüberliegenden Ende nicht vollständig geschlossen ist, können an der Stirnseite des Hauptkanals einzelne Absauglöcher durch die Innenoberfläche in den Hauptkanal einmünden, wobei diese Sauglöcher vorzugsweise die eingesaugte Flüssigkeit bereits auf jeweils einer Spiralbahn in den Hauptkanal einleiten und entsprechend spiralabschnittförmig ausgebildet sind. Auch wenn ein axiales Saugloch auf der Hauptachse in den Hauptkanal einmündet, prägen die Strömungsleiteinrichtungen der Strömung der abgesaugten Flüssigkeit vorzugsweise schon bei ihrem Durchtritt durch das Absaugloch die erfindungsgemäße Rotationskomponente auf.

Es versteht sich, dass der erfindungsgemäße Saugkopf vorteilhaft als Teil einer Absaugvorrichtung verwendet werden kann, wie sie aus der WO 2012/092948 A1 bekannt ist und bei der die Saugleistung abhängig von dem Signal eines Schallwellensensors gesteuert wird. Entsprechend kann der erfindungsgemäße Saugkopf auch einen solchen Schallwellensensor aufweisen.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einer Einspritzdüse für eine Hilfsflüssigkeit die Rede ist, ist dies so zu verstehen, dass genau eine Einspritzdüse, zwei Einspritzdüsen oder mehr Einspritzdüsen vorhanden sind. Die in den Patentansprüchen aufgeführten Merkmale können durch andere Merkmale ergänzt werden oder die einzigen Merkmale sein, die das jeweilige Erzeugnis aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt einen als solchen nicht unter die Patentansprüche fallenden Saugkopf in einem Längsschnitt längs seiner Hauptachse.
- **Fig. 2**: zeigt ein Detail eines weiteren als solchen nicht unter die Patentansprüche fallenden Saugkopfs in einem schematischen Schnitt senkrecht zu seiner Hauptachse.
- **Fig. 3 bis 5**: zeigen jeweils in einem Schnitt senkrecht zur Hauptachse ein Detail eines weiteren als solchen nicht unter die Patentansprüche fallenden Saugkopfs; und
- **Fig. 6 und 7**: erläutern eine spezielle zweiteilige Ausbildung eines Absauganschlusses bei einem erfindungsgemäßen Saugkopf in einem Längsschnitt längs der Hauptachse und einem Querschnitt quer zu der Hauptachse.

### FIGUREN BESCHREIBUNG

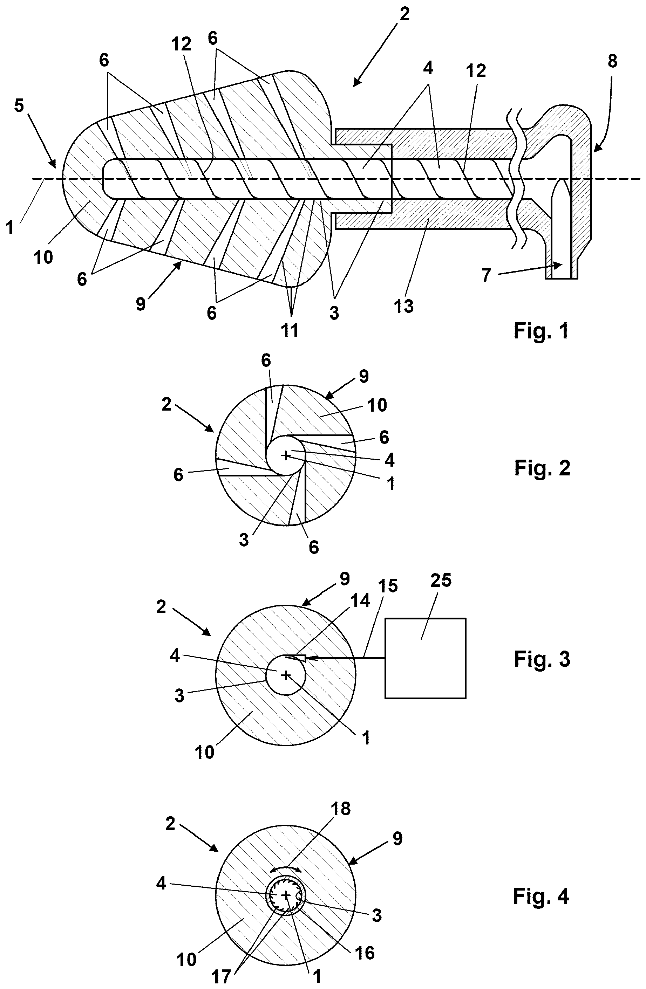

Ein in **Fig. 1** in einem Schnitt längs seiner Hauptachse 1 dargestellter Saugkopf 2 weist eine Innenoberfläche 3 auf, die einen sich längs der Hauptachse 1 erstreckenden Hauptkanal 4 begrenzt. An einem distalen Ende 5 des Saugkopfs 2 ist der Hauptkanal 4 geschlossen. In den Hauptkanal 4 münden mehrere Sauglöcher 6 ein. Ein Absauganschluss 7 ist an dem proximalen Ende 8 des Saugkopfs 2 an den Hauptkanal 4 angeschlossen. Der Absauganschluss 7 dient dazu, den Saugkopf 2 über eine Abscheideeinrichtung mit einer Unterdruckquelle zu verbinden, wobei die Abscheideeinrichtung dazu dient, eine mit dem Saugkopf 2 abgesaugte, organische Bestandteile umfassende Flüssigkeit abzuscheiden.

Die Sauglöcher 6 treten durch eine Außenoberfläche 9, die die äußeren Abmessungen des Saugkopfs 2 definiert, in den Saugkopf 2 ein. Von der Außenoberfläche 9 zu der Innenoberfläche 3 nehmen freie Querschnittsflächen der Sauglöcher 6 ab. Zudem nehmen die freien Querschnittsflächen der Sauglöcher 6 mit deren Abstand zu dem distalen Ende 5 hin ab. Das heißt, die Sauglöcher 6, welche dem Absauganschluss 7 am nächsten liegen, haben die kleinsten freien Querschnittsflächen. Zudem sind diese Sauglöcher 6 länger als die Sauglöcher 6, die näher an dem distalen Ende 5 liegen, weil ein Formkörper 10 des Saugkopfs 2, durch den hindurch die Sauglöcher 6 zu dem Hauptkanal 4 verlaufen, kegelstumpfförmige äußere Abmessungen aufweist. Auf diese Weise wird verhindert, dass dann, wenn nur die Sauglöcher 6 nahe dem distalen Ende 5 in eine abzusaugende Flüssigkeit eingetaucht werden, diese Sauglöcher 6 durch die anderen, Luft ansaugenden Sauglöcher 6 kurzgeschlossen werden.

Der Formkörper 10 kann beispielsweise durch 3D-Druck ausgebildet sein. Die Oberflächen des Formkörpers 10 sind mit einer zusammenhängenden glatten Beschichtung 11 versehen, die die Innenoberfläche 3 und die Außenoberfläche 9 ausbildet sowie die Sauglöcher 6 auskleidet. Die Beschichtung 11 kann insbesondere als Slipbeschichtung ausgebildet sein, die einen Überströmwiderstand für die abzusaugende Flüssigkeit stark herabsetzt.

Als Besonderheit des Saugkopfs 2 gemäß Fig. 1 sind Strömungsleiteinrichtungen vorgesehen, die einer Strömung der durch die Sauglöcher 6 in den Hauptkanal 4 eintretenden Flüssigkeit, die durch einen Unterdruck in dem Absauganschluss 7 hervorgerufen wird, innerhalb des Hauptkanals 4 eine Rotationskomponente um die Hauptachse 1 aufprägen. Das heißt, die Strömung durch die Hauptkanal 4 verläuft auf Spiralbahnen um die Hauptachse 1 durch den Hauptkanal 4. Dabei ist die Rotationskomponente dieser Strömung so groß, dass sich die abgesaugte Flüssigkeit in dem Hauptkanal 4 auch dann an die Innenoberfläche 3 anlegt und diese bedeckt, wenn neben der Flüssigkeit auch Luft durch die Sauglöcher 6 in den Hauptkanal 4 eingesaugt wird. Damit wird eine Vermischung dieser Luft mit der abgesaugten Flüssigkeit vermieden.

Zu den Bestandteilen der Strömungsleiteinrichtungen des Saugkopfs 2 in der Ausführungsform gemäß Fig. 1 zählen Verläufe der Sauglöcher 6 nicht direkt von der Seite auf die Hauptachse 1 zu, sondern zu dieser versetzt, spiralförmige Strömungsleitelemente 12 an der Innenoberfläche 3 und eine gegenüber der Hauptachse 1 versetzte seitliche Abzweigung des Absauganschlusses 7.

Der Saugkopf 2 gemäß Fig. 1 umfasst neben dem Formkörper 10 ein daran angeschlossenes Rohrstück 13, das länger oder kürzer als dargestellt sowie einstückig mit dem Formkörper 10 ausgebildet sein kann. Eine mehrteilige Ausbildung des Saugkopfs 2 kann jedoch dessen Reinigung und Sterilisation nach seiner Verwendung erleichtern.

Der in **Fig. 2** dargestellte Querschnitt senkrecht zu der Hauptachse 1 durch eine andere Ausführungsform des Saugkopfs 2 zeigt vier Sauglöcher 6, die jeweils eine von der Außenoberfläche 9 zu der Innenoberfläche 3 abnehmende freie Querschnittsfläche aufweisen und tangential zu einem Kreisbogen um die Hauptachse 1 durch die Innenoberfläche 3 in den Hauptkanal 4 einmünden. Hierdurch erhält die in den Hauptkanal 4 eingesaugte Flüssigkeit bereits in den Sauglöchern 6 einen Drall um die Hauptachse 1, so dass sich die Flüssigkeit längs der Hauptachse 1 auf den bereits angesprochenen Spiralbahnen durch den Hauptkanal 4 bewegt.

Der schematische Querschnitt durch eine weitere Ausführungsform des Saugkopfs 2 gemäß **Fig. 3** zeigt eine Einspritzdüse 14 für eine Hilfsflüssigkeit 15, beispielsweise ein flüssiges Antikoagulationsmittel, falls die abgesaugte Flüssigkeit Blut ist. Die Einspritzdüse 14 mündet tangential zu dem von der Innenoberfläche 3 beschriebenen Kreisbogen um die Hauptachse 1 in den Hauptkanal 4 ein. Mit einer Pumpe 25 durch die Einspritzdüse 14 in den Hauptkanal 4 injizierte Hilfsflüssigkeit 15 wird von der Innenoberfläche 3 umgelenkt, so dass sie sich längs Spiralbahnen um die Hauptachse 1 durch den Hauptkanal 4 bewegt, und nimmt dabei die in den Hauptkanal 4 angesaugte Flüssigkeit längs dieser Spiralbahnen mit.

Eine weitere aktive Maßnahme der Strömungsleiteinrichtung bei dem Saugkopf 2 ist in **Fig. 4** skizziert. Hier ist ein ringförmiger Beschleunigungskörper 16 in dem Formkörper 10 angeordnet.

Der Beschleunigungskörper 16 bildet zumindest teilweise die Innenoberfläche 3 aus, die den Hauptkanal 4 begrenzt. Dabei ist die Innenoberfläche 3 mit gegen die Umfangsrichtung um die Hauptachse 1 angestellten Schuppen 17 versehen. Konkret können diese Schuppen so gestaltet sein, wie um einen Lotuseffekt für die abzusaugende Flüssigkeit zu erzielen. Wenn jetzt der Beschleunigungskörper 6 wie durch einen Doppelpfeil 18 angedeutet um die Hauptachse 1 herum hin und her bewegt wird, wobei insbesondere seine Bewegung in Richtung der gewünschten Rotationskomponente der abgesaugten Flüssigkeit langsamer erfolgen kann als entgegen dieser Rotationskomponente, wird die abgesaugte Flüssigkeit von dem Beschleunigungskörper 16 in Richtung der gewünschten Rotationskomponente beschleunigt.

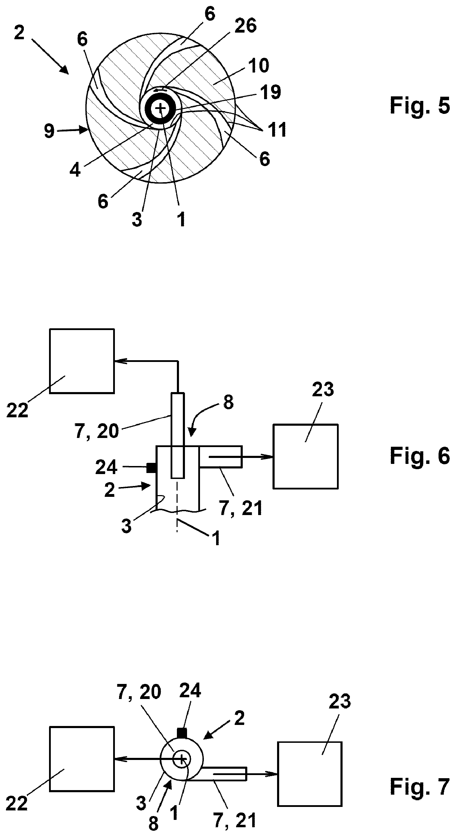

Auch die in **Fig. 5** illustrierte Ausführungsform des Saugkopfs 2 weist einen um die Hauptachse 1 drehangetriebenen Beschleunigungskörper 19 auf. Dieser Beschleunigungskörper 19 rotiert jedoch kontinuierlich in Richtung eines Drehpfeils 26 im Inneren des Hauptkanals 4. Die verbleibende freie Querschnittsfläche des Hauptkanals 4 zwischen der Innenoberfläche 3 und dem Beschleunigungskörper 19 ist ringförmig. Mit anderen Worten ist der Hauptkanal 4 um den Beschleunigungskörper 19 ein Ringkanal. In diesem Ringkanal wird der Strömung der abgesaugten Flüssigkeit mit dem rotierenden Beschleunigungskörper 19 eine Rotationskomponente in Richtung des Drehpfeils 26 aufgeprägt. Wenn dieser Beschleunigungskörper 19 mit einer schraubenförmigen Oberflächenstruktur versehen wird, kann er auch dazu dienen, die abgesaugte Flüssigkeit in Richtung der Hauptachse 1 zu fördern, d. h. die Strömung zu dem Absauganschluss hin zu unterstützen, oder sogar diese Strömung nach Art einer Absaugturbine originär hervorzurufen. Zudem zeigt Fig. 5 einen spiralförmigen Verlauf der Sauglöcher 6, die zugleich von der Außenoberfläche 9 zu der Innenoberfläche 3 abnehmende freie Querschnittsflächen aufweisen. Ein solcher Verlauf ist nur schwerlich durch ein abformendes oder spanabnehmendes Verfahren herstellbar. Der Formkörper 10, durch den die Sauglöcher 6 verlaufen, kann aber auch im 3D-Druck hergestellt und zur Verbesserung seiner Oberflächenqualität anschließend mit der glatten Beschichtung 11 versehen werden.

Die **Fig. 6 und 7** erläutern die Möglichkeit, am distalen Ende 8 eines erfindungsgemäßen Saugkopfs 2 mit Hilfe von zwei separaten Teilanschlüssen 20 und 21 des Absauganschlusses 7, die zu zwei separaten Unterdruckquellen 22 und 23 führen, mit der abgesaugten Flüssigkeit abgesaugte Luft einerseits und die abgesaugte Flüssigkeit andererseits getrennt abzuführen. Die Unterdruckquelle 22 saugt über den Teilanschluss 20 des Absauganschlusses 7 die sich im Bereich der Hauptachse 1 ansammelnde Luft ab, während die Unterdruckquelle 23 über den Teilanschluss 21 des Absauganschlusses 7 die sich an der Innenoberfläche 3 ansammelnde abgesaugte Flüssigkeit absaugt. Dabei kann ein Schallwellensensor 24 an dem Saugkopf 2 Luft und/oder Körperschallwellen erfassen. Abhängig von dem Signal des Schallwellensensors 24 können dann die Unterdruckquellen 22 und 23 gesteuert werden. Konkret kann dann, wenn auftretende Schallwellen darauf hinweisen, dass Luft in den Saugkopf 2 eingesaugt wurde, vornehmlich oder ausschließlich über den Teilanschluss 20 mit der Unterdruckquelle 22 abgesaugt werden. Wenn hingegen das Signal des Schallwellensensors 24 darauf hinweist, dass ausschließlich abzusaugende Flüssigkeit in den Saugkopf 2 abgesaugt wird, kann ausschließlich oder vornehmlich über den Teilanschluss 21 mit der Unterdruckquelle 23 abgesaugt werden. Bei dieser Vorgehensweise kann über den Teilanschluss 20 nicht nur reine Luft, sondern auch mit Luft aufgeschäumte abgesaugte Flüssigkeit abgesaugt werden, die sich aufgrund ihrer geringeren Dichte und der Rotationskomponente ihrer Strömung durch den Hauptkanal 4 ebenfalls im Bereich der Hauptachse 1 ansammelt und damit von der luftfreien Flüssigkeit an der Innenoberfläche 3 abtrennt. Wenn die abgesaugte Flüssigkeit Blut ist, findet sich zudem das "beste" Blut, d. h. gesunde Zellen eher in der äußeren Schicht an der Innenoberfläche 3, weil gesunde Erythrozyten eine höhere Masse als geschädigte Blutbestandteile aufweisen und sich aufgrund der von der Rotationskomponente der Strömung hervorgerufenen Zentrifugalkräfte außen ansammeln.

### BEZUGSZEICHENLISTE

- 1: Hauptachse
- 2: Saugkopf
- 3: Innenoberfläche
- 4: Hauptkanal
- 5: distales Ende
- 6: Saugloch
- 7: Absauganschluss
- 8: proximales Ende
- 9: Außenoberfläche
- 10: Formkörper
- 11: Beschichtung
- 12: Strömungsleitelement
- 13: Rohrstück
- 14: Einspritzdüse
- 15: Hilfsflüssigkeit
- 16: Beschleunigungskörper
- 17: Schuppe
- 18: Doppelpfeil
- 19: Beschleunigungskörper
- 20: Teilanschluss
- 21: Teilanschluss
- 22: Unterdruckquelle
- 23: Unterdruckquelle
- 24: Schallwellensensor
- 25: Pumpe
- 26: Drehpfeil

## Patentansprüche

1. Saugkopf (2) zum Absaugen einer organische Bestandteile umfassenden Flüssigkeit mit
- einer einen Hauptkanal (4) des Saugkopfs (2) definierenden Innenoberfläche (3), wobei sich der Hauptkanal (4) längs einer Hauptachse (1) des Saugkopfs (2) zu einem Absauganschluss (7) des Saugkopfs (2) hin erstreckt, und
- in den Saugkopf (2) eintretenden und durch die Innenoberfläche (3) in den Hauptkanal (4) einmündenden Sauglöchern (6),
wobei Strömungsleiteinrichtungen des Saugkopfs (2) derart ausgebildet sind, dass die Strömungsleiteinrichtungen einer durch einen Unterdruck in dem Absauganschluss (7) hervorgerufen Strömung der durch die Sauglöcher (6) eintretenden Flüssigkeit durch den Hauptkanal (4) eine Rotationskomponente um die Hauptachse (1) aufprägen,
**dadurch gekennzeichnet, dass** der Absauganschluss (7) einen auf der Hauptachse (1) an den Hauptkanal (4) anschließenden inneren Teilanschluss (20) und einen mit Abstand zu der Hauptachse (1) durch die Innenoberfläche (3) in den Hauptkanal (4) einmündenden äußeren Teilanschluss (21) aufweist.

2. Saugkopf (2) nach Anspruch 1, **dadurch gekennzeichnet, dass** die Strömungsleiteinrichtungen des Saugkopfs (2) derart ausgebildet sind, dass die Strömungsleiteinrichtungen der durch einen Unterdruck in dem Absauganschluss (7) hervorgerufen Strömung der durch die Sauglöcher (6) eintretenden Flüssigkeit durch den Hauptkanal (4) eine derartige Rotationskomponente um die Hauptachse (1) aufprägen, dass der von der Strömung der Flüssigkeit durch den Hauptkanal (4) zurückgelegte Weg aufgrund der Rotationskomponente um die Hauptachse (1) um mindestens 50 %, um mindestens 100 %, um mindestens 200 %, um mindestens 400 % oder um mindestens 900 % länger ist als die Erstreckung des Hauptkanals (4) längs der Hauptachse (1).

3. Saugkopf (2) nach einem der vorhergehenden Ansprüche, **dadurch gekennzeichnet, dass** die Strömungsleiteinrichtungen mindestens eines der folgenden Merkmale umfassen:
- Einmündungen der Sauglöcher (6) in den Hauptkanal (4) mit in Bezug auf einen Kreisbogen um die Hauptachse (1) tangentialer Richtungskomponente,
- eine Abzweigung des Absauganschlusses (7) von dem Hauptkanal (4) mit in Bezug auf einen Kreisbogen um die Hauptachse (1) tangentialer Richtungskomponente,
- eine Einmündung einer Einspritzdüse (14) für eine Hilfsflüssigkeit (15) in den Hauptkanal (4) mit in Bezug auf einen Kreisbogen um die Hauptachse (1) tangentialer Richtungskomponente,
- spiralförmige Strömungsleitelemente (12) an der Innenoberfläche (3),
- einen einen Teil der Innenoberfläche (3) ausbildenden, um die Hauptachse (1) herum hin und her drehangetriebenen Beschleunigungskörper (16),
- einen in dem Hauptkanal (4) angeordneten, um die Hauptachse (1) herum kontinuierlich drehangetriebenen Beschleunigungskörper (19).

4. Saugkopf (2) nach einem der vorhergehenden Ansprüche 1 bis 3, **dadurch gekennzeichnet, dass** die Strömungsleiteinrichtungen des Saugkopfs (2) derart ausgebildet sind, dass die Strömungsleiteinrichtungen der durch einen Unterdruck in dem Absauganschluss (7) hervorgerufen Strömung der durch die Sauglöcher (6) eintretenden Flüssigkeit durch den Hauptkanal (4) eine derartige Rotationskomponente um die Hauptachse (1) aufprägen, dass der von der Strömung der Flüssigkeit durch den Hauptkanal (4) aufgrund der Rotationskomponente um die Hauptachse (1) zusätzlich zurückgelegte Weg gegenüber der Erstreckung des Hauptkanals (4) längs der Hauptachse (1) zu dem Absauganschluss (7) hin zunimmt.

5. Saugkopf (2) nach einem der vorhergehenden Ansprüche 1 bis 3, **dadurch gekennzeichnet, dass** der von der Strömung der Flüssigkeit durch den Hauptkanal (4) aufgrund der Rotationskomponente um die Hauptachse (1) zusätzlich zurückgelegte Weg gegenüber der Erstreckung des Hauptkanals (4) längs der Hauptachse (1) zu dem Absauganschluss (7) hin abnimmt.

6. Saugkopf (2) nach einem der vorhergehenden Ansprüche, **dadurch gekennzeichnet, dass** ein freier Strömungsquerschnitt des Saugkopfs (2) längs der Strömung der Flüssigkeit zu dem Absauganschluss (7) hin einen stetigen Verlauf aufweist.

7. Saugkopf (2) nach einem der vorhergehenden Ansprüche, **dadurch gekennzeichnet, dass** die Sauglöcher (6) jeweils eine zu der Innenoberfläche (3) hin abnehmende freie Querschnittsfläche aufweisen.

8. Saugkopf (2) nach einem der vorhergehenden Ansprüche, **dadurch gekennzeichnet, dass** die freie Querschnittsfläche der Sauglöcher (6) jeweils von einer Außenoberfläche (9) des Absaugkopfs (2) bis zu der Innenoberfläche (3) um mindestens 50 % oder mindestens 67 % oder mindestens 75 % abnimmt.

9. Saugkopf (2) nach einem der vorhergehenden Ansprüche, **dadurch gekennzeichnet, dass** eines der Sauglöcher (6), das näher zu dem Absauganschluss (7) hin in den Saugkopf (2) eintritt, einen größeren Strömungswiderstand für die Flüssigkeit bis in den Hauptkanal (4) aufweist als eines der Sauglöcher (6), das weiter weg von dem Absauganschluss (7) in den Saugkopf (2) eintritt.

10. Saugkopf (2) nach Anspruch 9, **dadurch gekennzeichnet, dass** der Strömungswiderstand desjenigen der Sauglöcher (6), das zu dem Absauganschluss (7) hin am nächsten in den Saugkopf (2) eintritt, um mindestens 50 % oder um mindestens 100 % oder um mindestens 200 % größer ist als ein Strömungswiderstand desjenigen der Sauglöcher (6), das von dem Absauganschluss (7) am weitesten entfernt in den Saugkopf (2) eintritt.

11. Saugkopf (2) nach einem der vorhergehenden Ansprüche, **dadurch gekennzeichnet, dass** die Innenoberfläche (3) zumindest teilweise mit einer den Überströmungswiderstand für die Flüssigkeit herabsetzenden Slipbeschichtung versehen ist und/oder dass die Sauglöcher (6) mit einer den Überströmungswiderstand für die Flüssigkeit herabsetzenden Slipbeschichtung ausgekleidet sind.

12. Saugkopf (2) nach einem der vorhergehenden Ansprüche, **dadurch gekennzeichnet, dass** der Saugkopf (2) einen 3D-gedruckten Formkörper (10) umfasst, dessen Oberflächen mit einer zusammenhängenden glatten Beschichtung (11) versehen sind.

13. Saugkopf (2) nach einem der vorhergehenden Ansprüche, **dadurch gekennzeichnet, dass** eine Umschalteinrichtung, die zwischen den beiden Teilanschlüssen (20, 21) umschaltet, so ausgebildet ist, dass sie beim Ansaugen von Luft durch den Saugkopf (2) den auf der Hauptachse (1) an den Hauptkanal (4) angeschlossenen inneren Teilanschluss (20) öffnet und den mit Abstand zu der Hauptachse (1) durch die Innenoberfläche (3) in den Hauptkanal (4) einmündenden äußeren Teilanschluss (21) schließt.

14. Saugkopf (2) nach einem der vorhergehenden Ansprüche, **dadurch gekennzeichnet, dass** ein axiales Saugloch auf der Hauptachse (1) in den Hauptkanal (4) einmündet.

15. Saugkopf (2) nach einem der vorhergehenden Ansprüche, **dadurch gekennzeichnet, dass** der Hauptkanal (4) an seinem dem Absauganschluss (7) gegenüberliegenden Ende zumindest zu 33 % oder 50 % oder 67 % oder 75 % seiner freien Querschnittsfläche vor dem Ende geschlossen ist.

## Claims

1. Suction head (2) for suctioning a liquid containing organic components, comprising
- an inner surface (3) defining a main channel (4) of the suction head (2), the main channel (4) extending along a main axis (1) of the suction head (2) towards a suction connection (7) of the suction head (2), and
- suction holes (6) entering into the suction head (2) and feeding through the inner surface (3) into the main channel (4),
wherein flow-guiding devices of the suction head (2) are configured such that the flow-guiding devices impart a rotational component about the main axis (1) to a flow of the liquid through the main channel (4), the flow being brought about by a negative pressure in the suction connection (7), and the liquid entering through the suction holes (6),
**characterized in that** the suction connection (7) comprises an inner partial connection (20) connecting to the main channel (4) on the main axis (1), and an outer partial connection (21) feeding through the inner surface (3) into the main channel (4) at a distance to the main axis (1).

2. Suction (2) head according to claim 1, **characterized in that** the flow-guiding devices of the suction head (2) are configured such that the flow-guiding devices impart such a rotational component about the main axis (1) to the flow of the liquid through the main channel (4), the flow being brought about by a negative pressure in the suction connection (7) and the liquid entering through the suction holes (6), that a path covered by the flow of the liquid through the main channel (4), due to the rotational component about the main axis (1), is at least 50 %, at least 100 %, at least 200 %, at least 400 % or at least 900 % longer than the extension of the main channel (4) along the main axis (1).

3. Suction head (2) according to any of the preceding claims, **characterized in that** the flow guiding devices comprise at least one of the following features:
- the suction holes (6) feeding into the main channel (4) having a tangential direction component with respect to a circular arc around the main axis (1),
- the suction connection (7) branching off from the main channel (4) having a tangential direction component with respect to a circular arc around the main axis (1),
- an injection nozzle (14) for an auxiliary liquid (15) injecting into the main channel (4) having a tangential direction component with respect to a circular arc around the main axis (1),
- helical flow guiding elements (12) on the inner surface (3),
- an acceleration body (16) forming a part of the inner surface (3) and driven for rotation back and forth about the main axis (1),
- an acceleration body (19) arranged in the main channel (4) and continuously driven for rotation about the main axis (1).

4. Suction head (2) according to any of the claims 1 to 3, **characterized in that** the flow-guiding devices of the suction head (2) are configured such that the flow-guiding devices impart such a rotational component about the main axis (1) to the flow of the liquid through the main channel (4), the flow being brought about by a negative pressure in the suction connection (7) and the liquid entering through the suction holes (6), that the path, which is, due to the rotational component about the main axis (1), additionally covered by the flow of the liquid through the main channel (4) as compared to the extension of the main channel (4) along the main axis (1), increases towards the suction connection (7).

5. Suction head (2) according to any of the claims 1 to 3, **characterized in that** the path, which is, due to the rotational component about the main axis (1), additionally covered by the flow of the liquid through the main channel (4) as compared to the extension of the main channel (4) along the main axis (1), decreases towards the suction connection (7).

6. Suction head (2) according to any of the preceding claims, **characterized in that** a free flow cross-section of the suction head (2) comprises a steady course along the flow of the liquid towards the suction connection (7).

7. Suction head (2) according to any of the preceding claims, **characterized in that** each of the suction holes (6) comprises a free cross-sectional area that decreases towards the inner surface (3).

8. Suction head (2) according to any of the preceding claims, **characterized in that** the free cross-sectional area of each of the suction holes (6) decreases from an outer surface (9) of the suction head (2) up to the inner surface (3) by at least one of 50 % or at least 67 % or at least 75%.

9. Suction head (2) according to any of the preceding claims, **characterized in that** one of the suction holes (6) which enters into the suction head (2) closer to the suction connection (7) has a higher flow resistance for the liquid up into the main channel (4) than one of the suction holes (6) which enters into the suction head (2) further away from the suction connection (7).

10. Suction head (2) according to claim 9, **characterized in that** the flow resistance of that one of the suction holes (6) which enters into the suction head (2) closest to the suction connection (7) is at least 50 % or at least 100 % or at least 200 % higher than a flow resistance that one of the suction holes (6) which enters into the suction head (2) farthest away from the suction connection (7).

11. Suction head (2) according to any of the preceding claims, **characterized in that** the inner surface (3) is at least partially provided with a slip-coating reducing the overflow resistance for the liquid, and/or that the suction holes (6) are lined with a slip-coating reducing the overflow resistance for the liquid.

12. Suction head (2) according to any of the preceding claims, **characterized in that** the suction head (2) includes a 3D-printed shaped body (10) whose surfaces are provided with a continuous smooth coating (11).

13. Suction head (2) according to any of the preceding claims, **characterized in that** a switchover device which switches over between the two partial connections (20, 21) is configured such that it opens the inner partial connection (20) connected to the main channel (4) on the main axis (1) and closes the outer partial connection (21) feeding through the inner surface (3) into the main channel (4) at a distance to the main axis (1), when air is suctioned through the suction head (2).

14. Suction head (2) according to any of the preceding claims, **characterized in that** an axial suction hole feeds into the main channel (4) on the main axis (1).

15. Suction head (2) according to any of the preceding claims, **characterized in that** the main channel (4), at its end facing the suction connection (7), is closed by at least 33 % or at least 50 % or at least 67 % or at least 75 % of its free cross sectional area in front of said end.

## Revendications

1. Tête d'aspiration (2) pour l'aspiration d'un liquide comprenant des composants organiques avec
- une surface interne (3) définissant un canal principal (4) de la tête d'aspiration (2), dans lequel le canal principal (4) s'étend le long d'un axe principal (1) de la tête d'aspiration (2) en direction d'un raccord d'aspiration (7) de la tête d'aspiration (2) et
- des trous d'aspiration (6) entrant dans la tête d'aspiration (2) et débouchant à travers la surface interne (3) dans le canal principal (4),
dans lequel des dispositifs de guidage d'écoulement de la tête d'aspiration (2) sont conçus de sorte que les dispositifs de guidage d'écoulement confèrent à un écoulement, provoqué par une dépression dans le raccord d'aspiration (7), du liquide entrant par les trous d'aspiration (6) à travers le canal principal (4), une composante de rotation autour de l'axe principal (1),
**caractérisée en ce que** le raccord d'aspiration (7) comprend un raccord partiel interne (20) se raccordant sur l'axe principal (1) au canal principal (4) et un raccord partiel externe (21) débouchant, à une certaine distance de l'axe principal (1), à travers la surface interne (3), dans le canal principal (4).

2. Tête d'aspiration (2) selon la revendication 1, **caractérisée en ce que** les dispositifs de guidage d'écoulement de la tête d'aspiration (2) sont conçus de sorte que les dispositifs de guidage d'écoulement confèrent à un écoulement, provoqué par une dépression dans le raccord d'aspiration (7), du liquide entrant par les trous d'aspiration (6) à travers le canal principal (4), une composante de rotation autour de l'axe principal (1) telle que le trajet parcouru par l'écoulement du liquide à travers le canal principal (4), du fait de la composante de rotation autour de l'axe principal (1), est plus long d'au moins 50 %, d'au moins 100 %, d'au moins 200 %, d'au moins 400 % ou d'au moins 900 % par rapport à l'extension du canal principal (4) le long de l'axe principal (1).

3. Tête d'aspiration (2) selon l'une des revendications précédentes, **caractérisée en ce que** les dispositifs de guidage d'écoulement présentent au moins une des caractéristiques suivantes :
- embouchures des trous d'aspiration (6) dans le canal principal (4) avec une composante de direction tangentielle par rapport à un arc de cercle autour de l'axe principal (1),
- une bifurcation du raccord d'aspiration (7) du canal principal (4) avec une composante de direction tangentielle par rapport à un arc de cercle autour de l'axe principal (1),
- une embouchure d'une buse d'injection (14) pour un liquide auxiliaire (15) dans le canal principal (4) avec une composante de direction tangentielle par rapport à un arc de cercle autour de l'axe principal (1),
- des éléments de guidage d'écoulement (12) en forme de spirales sur la surface interne (3),
- un corps d'accélération (16) formant une partie de la surface interne (3) entraîné en rotation autour de l'axe principal (1) avec un mouvement de va-et-vient,
- un corps d'accélération (19) disposé dans le canal principal (4), entraîné en rotation de manière continue autour de l'axe principal (1).

4. Tête d'aspiration (2) selon l'une des revendications précédentes 1 à 3, **caractérisée en ce que** les dispositifs de guidage d'écoulement de la tête d'aspiration (2) sont conçus de sorte que les dispositifs de guidage d'écoulement confèrent à un écoulement, provoqué par une dépression dans le raccord d'aspiration (7), du liquide entrant par les trous d'aspiration (6) à travers le canal principal (4), une composante de rotation autour de l'axe principal (1) telle que le trajet supplémentaire parcouru par l'écoulement du liquide à travers le canal principal (4), du fait de la composante de rotation autour de l'axe principal (1), augmente par rapport à l'extension du canal principal (4) le long de l'axe principal (1) en direction du raccord d'aspiration (7).

5. Tête d'aspiration (2) selon l'une des revendications précédentes 1 à 3, **caractérisée en ce que** le trajet supplémentaire parcouru par l'écoulement du liquide à travers le canal principal (4), du fait de la composante de rotation autour de l'axe principal (1), diminue par rapport à l'extension du canal principal (4) le long de l'axe principal (1) en direction du raccord d'aspiration (7).

6. Tête d'aspiration (2) selon l'une des revendications précédentes, **caractérisée en ce qu'**une section transversale d'écoulement libre de la tête d'aspiration (2) présente, le long de l'écoulement du liquide vers le raccord d'aspiration (7), un tracé constant.

7. Tête d'aspiration (2) selon l'une des revendications précédentes, **caractérisée en ce que** les trous d'aspiration (6) présentent chacun une surface de section transversale libre qui diminue en direction de la surface interne (3).

8. Tête d'aspiration (2) selon l'une des revendications précédentes, **caractérisée en ce que** la surface de section transversale libre des trous d'aspiration (6) diminue respectivement d'une surface externe (9) de la tête d'aspiration (2) jusqu'à la surface interne (3) d'au moins 50 % ou d'au moins 67 % ou d'au moins 75 %.

9. Tête d'aspiration (2) selon l'une des revendications précédentes, **caractérisée en ce qu'**un des trous d'aspiration (6), qui entre dans la tête d'aspiration (2) plus près du raccord d'aspiration (7), présente une résistance à l'écoulement plus grande pour le liquide jusque dans le canal principal (4) qu'un des trous d'aspiration (6) qui entre dans la tête d'aspiration (2) plus loin du raccord d'aspiration (7).

10. Tête d'aspiration (2) selon la revendication 9, **caractérisée en ce que** la résistance à l'écoulement du trou d'aspiration (6) qui entre dans la tête d'aspiration (2) au plus près du raccord d'aspiration (7) est plus grande d'au moins 50 % ou d'au moins 100 % ou d'au moins 200 % qu'une résistance à l'écoulement du trou d'aspiration (6) qui entre dans la tête d'aspiration (2) au plus loin du raccord d'aspiration (7).

11. Tête d'aspiration (2) selon l'une des revendications précédentes, **caractérisée en ce que** la surface interne (3) est munie au moins partiellement d'un revêtement de glissement diminuant la résistance à l'écoulement pour le liquide et/ou **en ce que** les trous d'aspiration (6) sont revêtus d'un revêtement de glissement diminuant la résistance à l'écoulement pour le liquide.

12. Tête d'aspiration (2) selon l'une des revendications précédentes, **caractérisée en ce que** la tête d'aspiration (2) comprend un corps imprimé en 3D (10) dont les surfaces sont munies d'un revêtement (11) lisse et d'une seule pièce.

13. Tête d'aspiration (2) selon l'une des revendications précédentes, **caractérisée en ce qu'**un dispositif de commutation, qui commute entre les deux raccords partiels (20, 21), est conçu de façon à ouvrir le raccord partiel interne (20) raccordé sur l'axe principal (1) au canal principal (4) et à fermer le raccord partiel externe (21) débouchant à distance de l'axe principal (1) à travers la surface interne (3) dans le canal principal (4), lors de l'aspiration d'air à travers la tête d'aspiration (2).

14. Tête d'aspiration (2) selon l'une des revendications précédentes, **caractérisée en ce qu'**un trou d'aspiration axial débouche sur l'axe principal (1) dans le canal principal (4).

15. Tête d'aspiration (2) selon l'une des revendications précédentes, **caractérisée en ce que** canal principal (4) est fermé, au niveau de son extrémité opposée au raccord d'aspiration (7), au moins à hauteur de 33 % ou 50 % ou 67 % ou 75 % de sa surface de section transversale libre avant l'extrémité.
